# EUROPÄISCHE PATENTSCHRIFT

(11) **EP 0 531 954 B1**
(45) Veröffentlichungstag und Bekanntmachung des Hinweises auf die Patenterteilung: **05.06.1996**
(21) Anmeldenummer: 92115371.4
(22) Anmeldetag: 09.09.1992
(51) Int. Cl.: C02F 3/30

(54) **Verfahren und Vorrichtung zur C-Quelle Dosiersteuerung der Denitrifikationsstufe einer Wasserreinigungsanlage**
Process and apparatus for controlling the metering of carbon source in the denitrification step of a waste water purification plant
Procédé et dispositif pour la commande de dosage d'une source de carbone dans l'étage de dénitrification d'une installation de purification d'eau usée

(30) Priorität: 13.09.1991 DE 4130466
(43) Veröffentlichungstag der Anmeldung: 17.03.1993
(73) Patentinhaber: FORSCHUNGSZENTRUM JÜLICH GMBH, D-52425 Jülich (DE)
(72) Erfinder: Aivasidis, Alexander, Dr., W-5170 Jülich (DE)

(56) Entgegenhaltungen:
- EP-A- 0 396 057
- GB-A- 2 063 239
- US-A- 4 986 916
- CHEMICAL ABSTRACTS, vol. 95, no. 4, 27. Juli 1981, Columbus, Ohio, US; abstract no 29859j, seite 299. Spalte R
- CHEMICAL ABSTRACTS, vol. 86, no. 6, 7. Februar 1977, Columbus, Ohio, US; abstract no. 33743y, Seite 260 ;Spalte R ;

## Beschreibung

Die Erfindung bezieht sich auf ein Verfahren zur Dosiersteuerung der C-Quelle-Zugabe zur Denitrifikationsstufe einer Wasserreinigungsanlage, sowie auf eine Vorrichtung zur Durchführung des Verfahrens.

Die zunehmende Nitratbelastung auch des Grundwassers durch unkontrollierte Abgabe nitrathaltiger Abwässer in Oberflächenbereiche, insb. von hoch nitratbelasteten Industrieabwässern, wie solchen der Tierhaltung, Düngerproduktion und Nahrungsmittelindustrie, zwingt zunehmend zu einer Entfernung von Nitrit und Nitrat aus Abwässern mit bereits weitgehend abgebautem organischen Anteil bzw. solchen mit defizitärem C-Angebot. Man kennt für die Denitrifikation chemische Verfahren mit Ionenaustauschern, Membranverfahren oder katalytische Behandlungen, die jedoch insb. bei unterschiedlicher Abwasserzusammensetzung nicht befriedigen.

Favorisiert wird daher die biologische Denitrifikation, insb. die heterotrophe Denitrifikation, zur Beseitigung von oxidierten Stickstoffverbindungen aus Grundwasser oder Abwasser, die im Falle einer für den Erhalt der Denitrifikanten und als Elektronendonator für die Stickstoffreduktion unzureichenden C-Quelle der Zudosierung einer externen C-Quelle bedarf.

Als C-Quelle werden dabei neben (teuren) reinen Verbindungen wie Alkohol (insb. Methanol) oder Essigsäure auch Melasse oder Abfälle der Zuckerindustrie oder auch noch stark mit organischen Verbindungen belastetes Abwasser verwendet.

Bei einer solchen biologischen Denitrifikation ist einerseits eine ausreichende Entfernung des vorhandenen Nitrit/Nitrats wichtig, dessen Konzentration ggfs. erheblichen Schwankungen unterliegt. Andererseits sollen auch zu hohe C-Quelle-Zusätze vermieden werden, die eine erneute Belastung des Wassers darstellen würden, insb. aber im allgemeinen unökonomisch sind.

Es besteht daher ein erhebliches Interesse an einer angemessenen Dosiersteuerung, mit der eine verläßliche Entfernung von Nitrit und Nitrat sichergestellt und die Zugabe zu hoher C-Quelle-Überschüsse vermieden werden kann.

Bislang werden zu diesem Zweck kinetische Überlegungen angestellt (siehe D. Lompe u.a. in Chem.-Ing.-Tech. 63 (1991) Seiten 692-99), die auf der Kenntnis von Sättigungskoeffizienten und maximalen Wachstumsraten basieren und schwerlich als praxisnahe Dosiersteuerung zu handhaben sind.

Ziel der Erfindung ist daher ein praxisnahes Dosierkonzept, das auch ohne umfängliche Kenntnis von Systemparametern einsetzbar ist.

Das zu diesem Zweck entwickelte erfindungsgemäße Verfahren ist dadurch gekennzeichnet, daß man die Methankonzentration im Abgas der Denitrifikationsstufe überwacht und den C-Quelle-Zulauf derart steuert, daß die Methankonzentration im Abgas ≧ 0,03 %, insb. ≧ 0,2 % bleibt (die Gaskonzentrationen verstehen sich in Vol-%).
Dieses Verfahren geht von der Erkenntnis aus, daß eine im Wasser vorhandene C-Quelle grundsätzlich sowohl von Nitrit und Nitrat abbauenden denitrifizierenden Organismen als auch von methanbildenden methanogenen Mikroorganismen verwertet wird, die sich als Populationen in Wasserreinigungsanlagen generell finden. Da die Denitrifikation jedoch thermodynamisch gegenüber der Methanbildung begünstigt ist, wird die zugesetzte C-Quelle zunächst praktisch ausschließlich für die NOₓ⁻-Reduktion (Nitratatmung) verwendet. Erst nach weitgehender N-Elimination wird die Umwandlung der Kohlenstoffquelle durch die methanogenen Mikroorganismen zu Biogas merklich.

Daraus leitet sich ein praktikables Regelungskonzept für die C-Quelle-Zufuhr ab, das darauf basiert, daß im Abgas der Dentrifikation auftretende deutlich meßbare Methankonzentrationen ein verläßlicher Hinweis auf die ausreichende Denitrifikation im behandelten Abwasser sind, da die Methanbildung erst nach ausreichender Denitrifizierung von vorhandenem Nitrit und Nitrat im Wasser umfänglich wird, die Methan-Löslichkeit im Wasser gering ist und Methan bei der globalen Gasfreisetzung im System rasch in den Gasraum gelangt (dessen Volumen generell als klein gegen das Flüssigkeitsvolumen angenommen werden kann) und mit dem Abgasstrom abgeleitet wird.

Dabei werden Werte unter 0,03 % Methan im Abgas als kritisch angesehen. Vorzugsweise wird jedoch dafür gesorgt, das Methankonzentrationen zwischen 0,2 und 0,4 %, insb. zwischen 0,2 und 0,3 % im Abgas der Denitrifikation beständig nachweisbar sind. Abweichungen von diesen Werten werden dann (je nach Richtung) als Signal zur Steigerung oder Verminderung des C-Quelle-Zulaufs verwendet.

Der Methan-Signalgeber kann unmittelbar im Gasraum über der Flüssigkeit der Denitrifikation vorgesehen werden, zweckmäßig ist jedoch eine gesonderte Meßzelle, durch die kontinuierlich ein gewisser Abgasstrom geleitet wird.

Selbstverständlich sind auch noch höhere Methangehalte zuzulassen, wenn durch einen dem entsprechenden, hohen Überschuß an C-Quelle ein höheres Maß an Sicherheit eingekauft werden soll.

Zusätzlich kann das Redoxpotential im Abwasser der Denitrifikationstufe überwacht und damit ein die unmittelbar im Reaktor vorliegenden Verhältnisse wiedergebender Wert gemessen werden: Bei ungenügender C-Quelle-Konzentration im Wasser steigt das Redoxpotential in der Flüssigkeit an, wobei Werte zwischen -90 bis -100 mV als kritisch angesehen werden können. Üblicherweise wird das Redoxpotential im Bereich von -340 bis -470 mV behalten.

Das erfindungsgemäße Verfahren ist insb. nützlich für die Denitrifikationsstufe in Industrieabwasserreinigungsanlagen, es ist jedoch auch bei der Denitrifikation im Rahmen den Trinkwasseraufbereitung anwendbar.

Eine Vorrichtung zur Durchführung des Verfahrens umfaßt im wesentlichen eine mit dem Gasraum der Denitrifikation verbundene Methanmeßeinrichtung und einen Dosierregler in der C-Quelle-Zuleitung zur Denitrifikationsstufe, der den Zulauf proportional zu der von der Methanmessung ermittelten Sollwertabweichung steuert.

Nachfolgend wird die Erfindung anhand von Beispielen beschrieben, bei denen als C-Quelle Methanol zudosiert wurde. Diese Beschreibung nimmt Bezug auf die angefügten Zeichnungen. Es zeigen schematisch:
- Fig. 1: eine Laboranordnung zur erfindungsgemäßen Dosiersteuerung;
- Fig. 2 u.3: Fließschemen für eine denitrifizierende Abwasserbehandlung mit Methanol-Dosiersteuerung und
- Fig. 4: den zeitlichen Verlauf von Methanoldurchfluß, CH₄-Abgaskonzentration und Redoxpotential in der Denitrifikationsstufe.

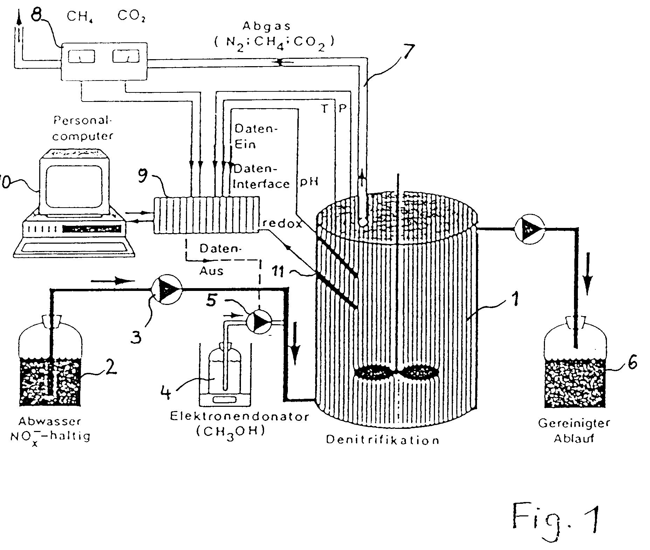

In Figur 1 ist ein Fließschema für einen Denitrifikationsprozeß mit rechnergestützter On-line-Datenerfassung und Regelung der Elektronendonator-Zugabe bei der Stickstoffelimination in Abwässern mit Kohlenstoffdefizit (unterstöchiometrischem C/N-Verhältnis) dargestellt.

In den Denitrifikationsreaktor 1 wird NOₓ⁻-haltiges, C-armes Abwasser aus 2 über die Pumpe 3 eingespeist, dem Methanol aus einem Vorrat 4 über eine gesteuerte Dosierpumpe 5 zugemischt wird. Der Ablauf gelangt in den Nachreiniger 6. Ein Abgasstrom 7 läuft über die CO₂- und die CH₄-Anzeige 8, deren Meßwerte über das Interface 9 zum Personal Computer 10 geschickt werden, der Steuerimpulse für die Methanol-Dosierung über das Interface 9 an die Dosierpumpe 5 liefert. Unmittelbar in der Reaktor-Flüssigkeit oder im Ablauf wird mit der Sonde 11 das Redoxpotential ermittelt und ein entsprechendes Signal an das Interface 9 abgegeben. Dieses erhält auch Signale für T, p und pH für eine ergänzende Prozeßsteuerung.

Bei Untersuchungen mit dieser Anordnung gemäß Figur 1 über die Denitrifikation von kohlenstoffarmem nitrathaltigen Abwasser (mit 0,3 bis 3 g NO₃⁻-N pro Liter) unter Zugabe von Methanol als C-Quelle konnte eine eindeutige Korrelation zwischen Nitratatmung und Methanogenese festgestellt werden: Erst nach weitgehender N-Elimination wurde die Kohlenstoffquelle durch die methanogenen Mikroorganismen merklich zu Biogas umgewandelt , wobei dann, je nach Überschuß, Methankonzentrationen von bis zu 70 % gemessen wurden.

Dieser Prozeßablauf behält auch seine Gültigkeit bei, wenn nicht direkt methanisierbare Elektronendonatoren zum Einsatz kommen oder bereits im Rohabwasser vorliegen. In solchen Fällen wird das dann maßgebliche methanogene Substrat, Essigsäure, durch die fermentative und acetogenetische Aktivität vorgelagerter Bakteriengruppen aus komplexen organischen Molekülen gebildet und dient den in Frage kommenden Mikroorganismenarten als Substrat. D.h., an Stelle von Methanol konnten andere C-Lieferanten wie z.B. Molke, Brauereiabwässer und dergleichen in angemessenen Mengen zudosiert werden.

Methanol wird lediglich wegen seiner vollständigen Umsetzung zu CO₂, guten Wasserlöslichkeit und der Möglichkeit weitgehend stöchiometrischer Dosierung vielfach bevorzugt, wobei gemäß der chemischen Gleichung für die Denitrifizierung von Nitrat mit Methanol-Zugabe für die Reduktion von 1 g NO₃-N unter Berücksichtigung der Überschußschlammbildung stöchiomtetrisch 2,47 g Methanol erforderlich sind.

Für die methan-abhängige Regelungsstrategie der Elektronendonatorzugabe wurden Regelkreise vorgesehen, deren Sollwerte für den entsprechenden Parameter (Dosierung des Elektronendonators) nach dem Kriterium der Minimierung der Methankonzentration über eine Set-point-control-Regelung mit Hilfe eines Personal Computers vorgegeben wurden.

Als eine weitere, komplementäre Führungsgröße wurde neben der Methankonzentration im Abgas der Denitrifikation auch das Redoxpotential im Ablauf der Denitrifikationsstufe herangezogen: Bei einer Akkumulation von NOₓ⁻ im Ablauf stellt sich ein Redoxpotential von -50 bis -110 mV ein, während bei vollständiger Denitrifikation und beginnender Methanogenese - entsprechend dem Optimalzustand des dargestellten Regelkonzeptes - stark negative Redoxpotentialwerte in der Größenordnung von -340 bis -430 mV bzw. bis herauf zu 470 mV gemessen werden. Der Elektronendonator-Überschuß ist dann ≦ 1%.

Somit kann über die Führungsgröße "Redoxpotential" ein massiver Einbruch von NOₓ⁻ rechtzeitig identifiziert werden.

Durch kombinierte Verwendung von direkt meßbaren Werten und den daraus ermittelten zeitlichen Ableitungen dieser Größen wird eine Entscheidungsmatrix aufgebaut, wonach der Rechner den extern vorgebbaren Sollwert für den Regelkreis der Dosierpumpe auch unter Berücksichtigung der ermittelten Trends verstellt.

Ein optimaler Betriebszustand der Denitrifikation ist gegeben, wenn die Methankonzentration zwischen 0,2 bis 0,3 % liegt und die positive Änderungsgeschwindigkeit derselben ≦ 250 ppm/h beträgt. Unter diesen Bedingungen konnte stets eine weitgehende Denitrifikation beobachtet werden, wobei das Redoxpotential automatisch Werte von -340 bis -430 mV annimmt.

Höhere positive bzw. negative Änderungsgeschwindigkeiten der Methankonzentration werden gemäß dem negativen Rückkoppelungsprinzip, welches einem Regelkreis in immanenter Weise zugrundeliegt, durch eine entsprechende Änderung der Dosierleistung für den Elektronendonator beantwortet. Die Höhe dieser Änderung sowie der Zeitpunkt der Eingriffnahme (sofortiger bzw. erst nach Ablauf einer angemessenen Wartezeit nach der vorangegangenen Verstellung) richtet sich sowohl nach der Größe der Ist-Wert-Abweichung vom definierten Sollwert für den Steadystate als auch nach der Änderungsgeschwindigkeit der Meßgröße.

So wird z.B. bei genügender positiver Abweichung vom Sollwert und entsprechender großer positiver Änderungsgeschwindigkeit der Methankonzentration eine entsprechend kräftige Reduzierung der Dosiergeschwindigkeit für den Elektronendonator vorgenommen. Entsprechend wird im Falle einer negativen Abweichung vom Sollwert (z.B. [CH₄ %] < 0,2 %) und einer raschen Abnahme der Methankonzentration durch eine starke Erhöhung des Massenflusses für den Elektronendonator begegnet.

Bei der Durchführung der Methan-Konzentrationsmessung ist insbesondere darauf zu achten, daß die Probennahme an einer repräsentativen Stelle der Reaktorgasphase erfolgt und möglichst ohne nennenswerte Totzeiten dem On-line-Analysator zugeführt wird. Tabelle 1 beinhaltet als Beispiel ein mögliches Muster der zu implementierenden Algoritmenmatrix für die rechnergestützte Regelung der Elektronendonatorzugabe (Methanol) mit Hilfe der on-line gemessenen CH₄-Konzentration im Abgas der Denitrifikationsstufe. Aufgrund der Komplexität des Verhaltens biologischer Systeme sowie der verhältnismäßig langen Zeitkonstanten gegenüber beispielsweise chemisch-katalytischen Prozessen (mit Verweilzeiten von Sekunden bis Minuten bei chemischen jedoch Stunden bis Tagen bei biologischen Systemen) kann eine derartige Regelungsaufgabe durch herkömmliche PI- bzw. PID-Regler nur mit einiger Ungenauigkeit bewältigt werden. Daher wird hier der Einsatz von Prozeßrechnern bevorzugt, wo über eine entsprechende Software eine auf den Prozeß zugeschnittene Algoritmenmatrix definiert und zur Prozeßregelung herangezogen wird. Tabelle 1 zeigt als Beispiel ein Datenwerk von Entscheidungs-Kriterien, das natürlich den jeweiligen Prozeßanforderungen anzupassen ist und in Kommunikation mit dem jeweiligen Prozeß zu erarbeiten und für den jeweiligen Einzelfall individuell zu optimieren ist.

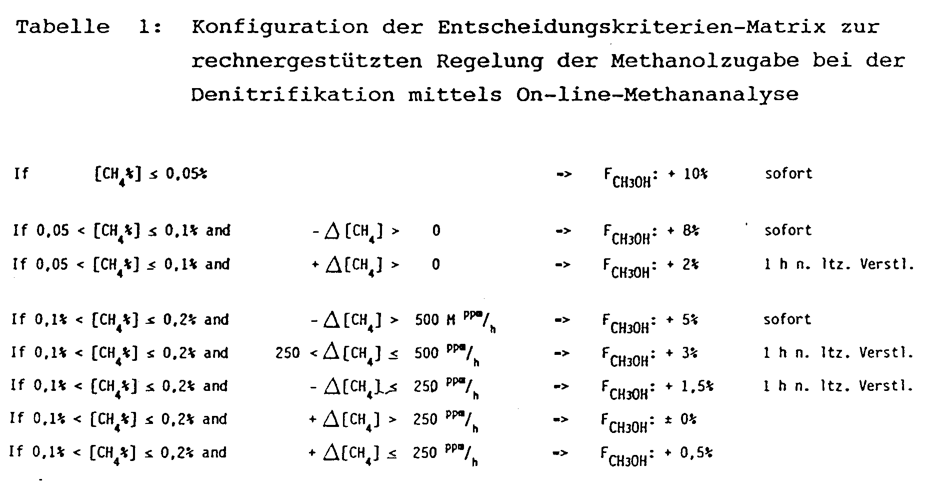

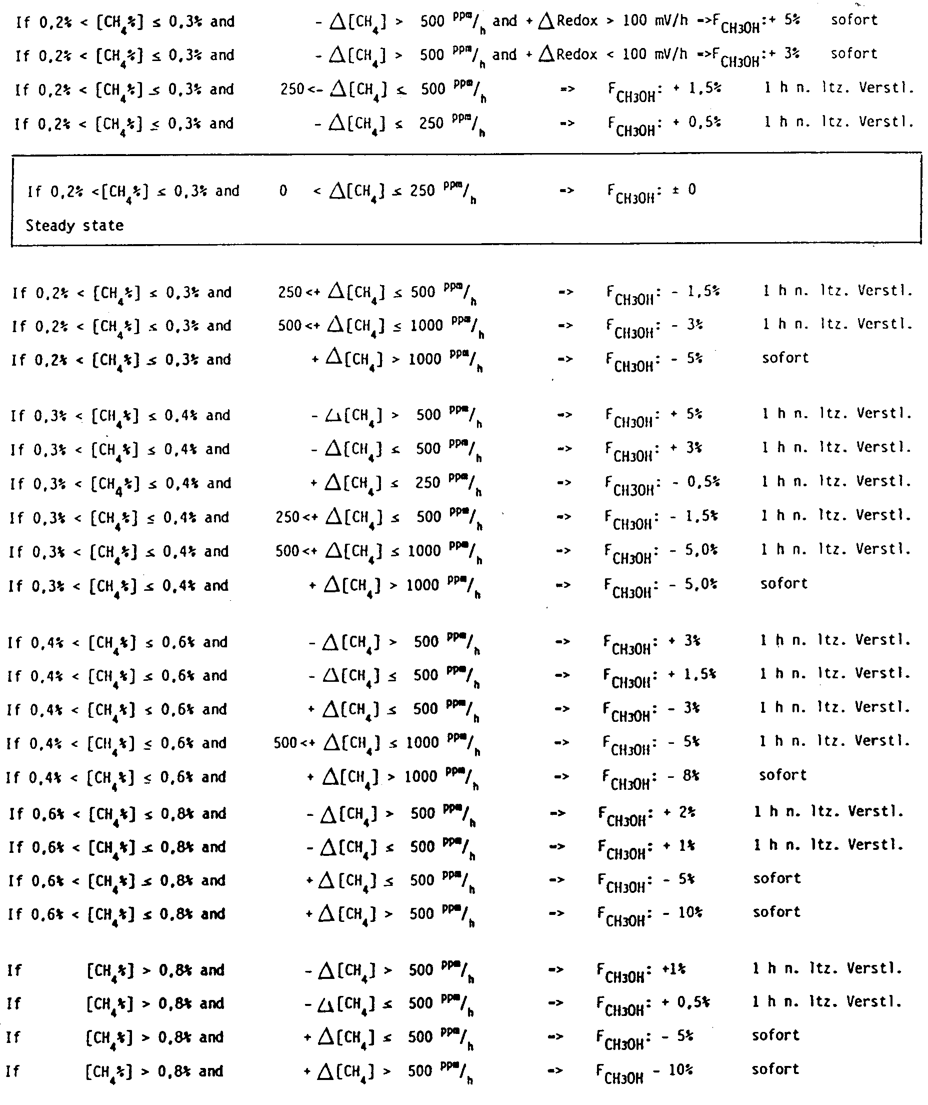

Die erfindungsgemäße Dosiersteuerung ist generell für industrielle, landwirtschaftliche und kommunale Abwässer nützlich, die überlicherweise erhebliche Mengen Ammoniumstickstoff erhalten, dessen biologische Elimination in zwei Schritten abläuft:

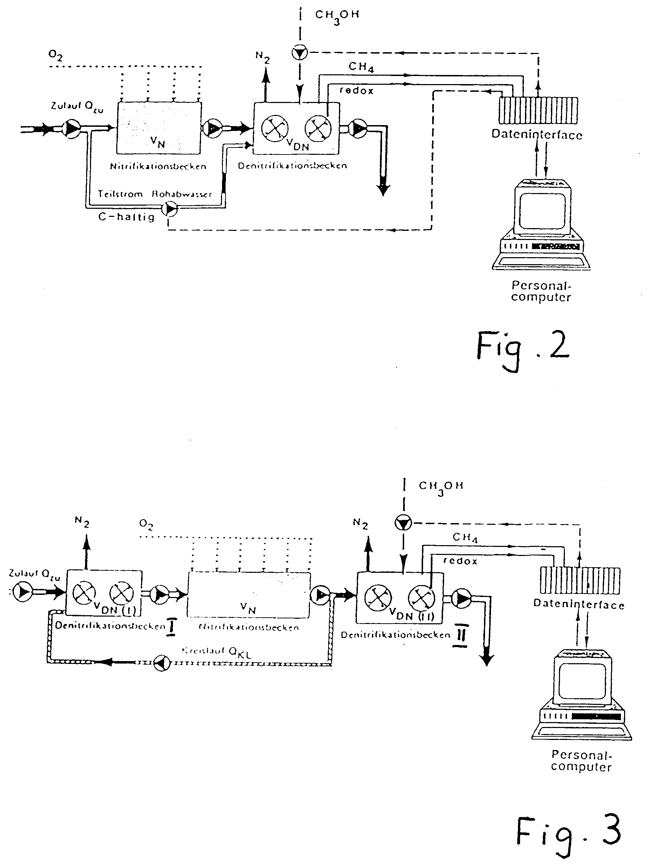

Zunächst wird unter oxischen (aeroben) Bedingungen Ammonium durch Mikroorganismen der Gattung Nitrosomonas und Nitrobacter zu Nitrit und Nitrat nitrifiziert. In einem anschließenden anoxischen (anaeroben) Denitrifikationssschritt werden dann die gebildeten Oxidationsprodukte durch heterotrophe Nitratatmer (Denitrifikanten) bei gleichzeitiger Verwertung einer Kohlenstoffquelle als Elektronendonator zu molekularem Stickstoff (N₂) reduziert (siehe auch Figur 2).

Gemäß Figur 2 wird das erfindungsgemäße Prinzip für die By-pass-Regelung eines Rohabwasser-Teilstroms angewandt, der unter Umgehung der Nitrifikationsstufe als C-haltiges Medium direkt der Denitrifikationsstufe zugeführt wird. Da ein Überschuß zu einer signifikanten CSB-Höherbelastung sowie Ammoniumakkumulation im Ablauf der Gesamtanlage führen würde, wird auch hier eine exakte Mengendosierung vorgesehen. Figur 2 zeigt ferner eine Regelung für eine C-Quellen-Supplementierung in Form von Methanol.

Bei ausreichend organisch belasteten Abwässern ist aber auch eine vorgeschaltete Denitrifikation mit Rücklauf aus der Nitrifikationsstufe möglich, wie in Figur 3 dargestellt ist.

Bei dem gezeigten Beispiel wird nun vorausgesetzt, daß eine C-Quelle-Zudosierung in der vorgeschalteten Dentitrifikationsstufe I überflüssig ist, während in der nachgeschalteten Denitrifikationsstufe II eine Methanolzulaufsteuerung gemäß der Erfindung vorgesehen wird. Dabei kann die externe C-Quelle (CH₃OH) allein oder zusamen mit einem Teilstrom des Rohabwassers als Elektronendonator angewandt werden.

Eine vorgeschaltete Denitrifikation (siehe Figur 3, linke Hälfte) wird inbesondere dann sinnvollerweise angewandt, wenn ein deutlicher Kohlenstoffüberschuß gegenüber der Stickstoffkonzentration vorliegt. Aufgrund der Vorteile, welche mit der vorgeschalteten Denitrifikation zusammenhängen, wird diese Verfahrensvariante allerdings auch vielfach bei ungünstigeren C/N-Verhältnissen realisiert. Die Regelung eines dann zu supplementierenden externen Elektronendonators (nicht ausgeführt) kann ebenfalls erfindungsgemäß erfolgen. Bei diesem Konzept wird allerdings ein sehr hoher Rücklaufstrom von der nachgeschalteten Nitrifikationsstufe zum Eingang der Anlage notwendig. Durch die gleichzeitige hohe hydraulische Belastung in der Nitrifikationsstufe wird die biologische Aktivität durch teilweises Auswaschen der Mikroorganismen beeinträchtigt, so daß zur Vermeidung einer Ammoniumakkumulation im Ablauf ein beträchtliches Nitrifikationsvolumen bereitgestellt werden muß.

Zweckmäßig ist daher eine kombinierte Betriebsweise, bei der gerade soviel Nitrat-Stickstoff aus dem Ablauf der Nitrifikationsstufe über den Rücklauf zur Eingangsdenitrifikation gelangt, daß entsprechend dem Angebot an C-Quelle lediglich partiell denitrifiziert wird (z.B. ca. 40 %). Der dann im Ablauf der Nitrifikation vorliegende Stickstoff wird einer weiteren nachgeschalteten Denitrifikationsstufe zugeleitet, wo durch Zugabe eines externen Elektronendonators weitgehend denitrifiziert wird (s. Figur 3).

Den charakteristischen Aufbau einer optimierten Matrixkonfiguration, welche sich aus der Modifikation der in Tabelle 1 dargestellten Kriterien ableitet, zeigt Tabelle 2.

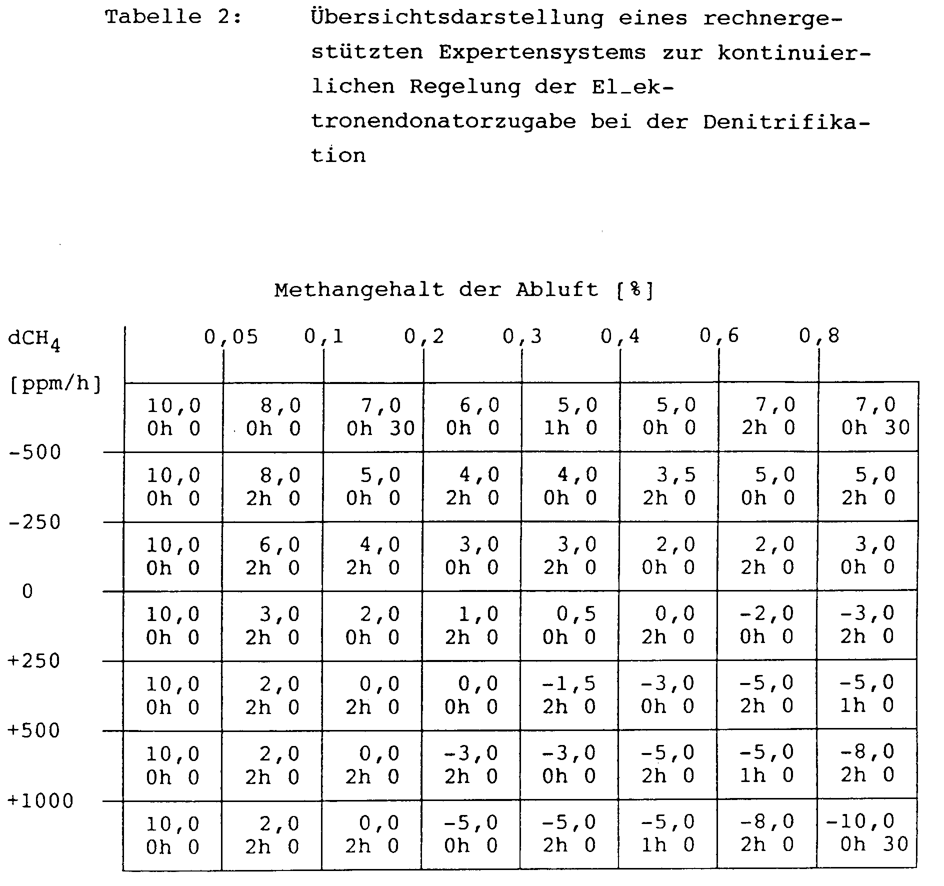

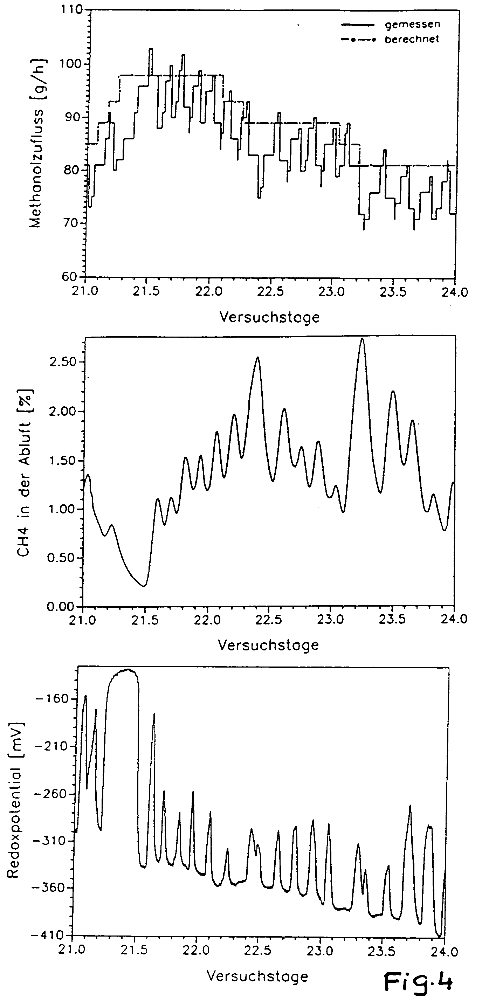

Diese wurde bei einem Experiment eingesetzt, bei dem der Nitratdurchfluß stufenweise jeweils um 5 % und einem zeitlichen Abstand zwischen den Verstellungen von zwei Stunden (d.h. um insgesamt 20 % innerhalb von 6 Stunden) erhöht wurde. Nachdem dieser über Nacht aufrechterhalten wurde, erfolgte am darauffolgenden Tag eine zweimalige Erniedrigung um jeweils 5 % und am Tag darauf eine ebenfalls identische zweistufige Senkung des Nitrat-Zulaufstroms und Rückführung auf den zu Beginn der Messung eingestellten Durchflußwert. Die in Fig. 4 dargestellten Ergebnisse sprechen für sich: Es kommt weder zu einem Überschwingen der CH₄-Konzentration noch zu einem "Außer-Kontrolle-Geraten" des Prozesses. Sowohl eine anfangs stärkere Abnahme der Methankonzentration in der Abluft als auch ein nach Reduzierung des Nitrat-Zulaufstromes rapider Konzentrationsanstieg werden glatt und problemlos aufgefangen. Die höchsten positiven Redoxpotentialwerte liegen in der Größenordnung von -130 mV. Zu keinem Zeitpunkt während der Experimentdurchführung trat Nitrat und Methanol im Ablauf der Denitrifikationsanlage auf.

Diese Regelungsstrategie führt auch darüberhinaus zu einer signifikanten Einsparung der erforderlichen Elektronendonatorsupplementierung auf das absolut notwendige (minimale) Maß, indem sowohl sonstige im Abwasserzulauf vorliegende Elektronendonatoren als auch eine integrierte Verwertung von Lysisproduktion aus Biomasse ebenfalls berücksichtigt werden. Aus der Betrachtung der Ganglinien von stöchiometrisch erforderlichem und tatsächlichem Methanoldurchfluß ergibt sich, daß die vom Regler eingestellten Methanoldurchflüsse in allen Fällen deutlich geringer sind und dabei der durchschnittliche Methanol-Minderbedarf beachtliche 10 % beträgt.

## Patentansprüche

1. Verfahren zur Dosiersteuerung der C-Quelle-Zugabe zur Denitrifikationsstufe einer Wasserreinigungsanlage,
**dadurch gekennzeichnet,**
daß man die Methankonzentration im Abgas der Denitrifikationsstufe überwacht und den C-Quelle-Zulauf derart steuert, daß die Methankonzentration im Abgas ≧ 0,03 %, insb. ≧ 0,2 % bleibt.

2. Verfahren nach Anspruch 1,
**dadurch gekennzeichnet,**
daß man den Zulauf derart steuert, daß die Methankonzentration innerhalb von 0,2 - 0,4 %, inbs. 0,2 - 0,3 % bleibt.

3. Verfahren nach Anspruch 1 oder 2,
**dadurch gekennzeichnet,**
daß man zusätzlich das Redoxpotential im Abwasser der Denitrifikationsstufe überwacht und ggf. durch zusätzliche C-Quelle-Zulaufsteuerung bei Werten im Bereich von -340 bis -470 mV hält.

4. Verfahren nach Anspruch 3,
**dadurch gekennzeichnet,**
daß der C-Quelle-Zulauf spontan freigegeben wird, wenn das Redoxpotential -100 mV übersteigt.

5. Verfahren nach einem der vorangehenden Ansprüche,
**gekennzeichnet durch**
die Anwendung bei der Denitrifikation einer Industrieabwasserreinigungsanlage.

6. Vorrichtung zur Dosiersteuerung der C-Quelle-Zugabe zu einer Denitrifikationsstufe einer Wasserreinigungsanlage,
**gekennzeichnet durch**
eine mit dem Gasraum der Denitrifikation verbundene Methanmeßeinrichtung und durch einen Dosierregler in der C-Quelle-Zuleitung zur Denitrifikationsstufe, der den Zulauf proportional zu der von der Methanmessung ermittelten Sollwertabweichung steuert.

7. Vorrichtung nach Anspruch 6,
**gekennzeichnet durch**
eine On-line Methanmessung und einen PID-Regler oder insb. einen Prozeßrechner mit Datenwandler für die Zulaufsteuerung der C-Quelle.

8. Vorrichtung nach Anspruch 6 oder 7,
**gekennzeichnet durch**
eine mit dem Dosierregler verbundene, im Abwasser der Denitrifikationsstufe angeordnete Redoxpotentialmeßsonde, deren Signal zum Dosierregler als zusätzlich Steuergröße für den C-Quelle-Zulauf geleitet wird und einen erhöhten Methanolzulauf sowie ggfs. ein Alarmsignal im Falle eines steilen Redoxpotentialanstiegs über -340mV hinaus auslöst.

## Claims

1. Method for dosage control of the C-source addition to the denitrification stage of a water purification plant, characterized in that the methane concentration in the denitrification stage exhaust gas is monitored and the C-source feed is controlled in such a manner that the methane concentration in the exhaust gas remains ≧0.03%, in particular ≧0.2%.

2. Method according to Claim 1, characterized in that the feed is controlled in such a manner that the methane concentration remains within 0.2-0.4%, in particular 0.2-0.3%.

3. Method according to Claim 1 or 2, characterized in that the redox potential in the denitrification stage wastewater is additionally monitored and, if appropriate, is maintained by additional C-source feed control at values in the range from -340 to -470 mV.

4. Method according to Claim 3, characterized in that the C-source feed is spontaneously opened, if the redox potential exceeds -100 mV.

5. Method according to one of the preceding claims, characterized by the use in the denitrification of an industrial wastewater purification plant.

6. Apparatus for dosage control of the C-source feed to a denitrification stage of a water purification plant, characterized by a methane measuring device connected to the gas space of the denitrification and by a dosage controller in the C-source feed line to the denitrification stage which controls the feed in proportion to the deviation from the set point determined from the methane measurement.

7. Apparatus according to Claim 6, characterized by an on-line methane measurement and a PID controller or, in particular, a process control computer with a data converter for controlling the feed of a C-source.

8. Apparatus according to Claim 6 or 7, characterized by a redox potential measuring probe arranged in the denitrification stage wastewater and connected to the dosage controller, the signal of which measuring probe is passed to the dosage controller as an additional control variable for the C-source feed and triggers an increased methanol feed or, if appropriate, an alarm signal in the event of a steep increase in redox potential above -340 mV.

## Revendications

1. Procédé pour la commande de dosage d'une addition d'une source de carbone pendant l'opération de dénitrification dans une installation de purification de l'eau, caractérisé en ce qu'on surveille la concentration de méthane dans les effluents gazeux de l'opération de dénitrification et on commande l'amenée de la source de carbone d'une manière telle que la concentration de méthane dans les effluents gazeux reste ≧ 0,03 %, en particulier ≧ 0,2 %.

2. Procédé selon la revendication 1, caractérisé en ce qu'on commande l'amenée d'une manière telle que la concentration de méthane reste comprise entre 0,2 et 0,4 %, en particulier entre 0,2 et 0,3 %.

3. Procédé selon la revendication 1 ou 2, caractérisé en ce qu'on surveille, en outre, le potentiel d'oxydoréduction dans les eaux usées pendant l'opération de dénitrification et éventuellement, on le maintient à des valeurs comprises entre -340 et -470 mV grâce à une commande supplémentaire pour l'amenée de la source de carbone.

4. Procédé selon la revendication 3, caractérisé en ce que l'amenée de la source de carbone est libérée spontanément quand le potentiel d'oxydoréduction dépasse -100 mV.

5. Procédé selon l'une des revendications précédentes, caractérisé en ce qu'on utilise une installation de purification des eaux usées industrielles pour la dénitrification.

6. Dispositif pour la commande de dosage d'une addition d'une source de carbone pendant l'opération de dénitrification dans une installation de purification de l'eau, caractérisé par un système de mesure du méthane relié à l'espace gazeux pour la dénitrification et par un régulateur de dosage dans la conduite d'amenée de la source de carbone pour l'opération de dénitrification, qui commande l'amenée proportionnellement à l'écart entre la mesure de méthane déterminée et la valeur de consigne.

7. Dispositif selon la revendication 6, caractérisé par un dispositif de mesure de méthane monté en série et un régulateur PID ou en particulier un ordinateur de processus avec un convertisseur de données pour la commande d'amenée de la source de carbone.

8. Dispositif selon la revendication 6 ou 7, caractérisé par une sonde de mesure du potentiel d'oxydoréduction, reliée au régulateur de dosage et disposée dans les eaux usées de l'opération de dénitrification, dont le signal est amené au régulateur de dosage en tant que valeur de commande supplémentaire pour l'amenée de la source de carbone et déclenche une augmentation de l'amenée de méthanol, ainsi qu'éventuellement un signal d'alarme dans le cas d'une forte augmentation du potentiel d'oxydoréduction au-delà de -340 mV.
